(19) Europäisches Patentamt
European Patent Office
Office européen des brevets

(11) **EP 3 122 324 B1**

(12) **FASCICULE DE BREVET EUROPEEN**

(45) Date de publication et mention
de la délivrance du brevet:
**03.10.2018 Bulletin 2018/40**

(21) Numéro de dépôt: **15713462.8**

(22) Date de dépôt: **27.03.2015**

(51) Int Cl.:
*A61K 8/365* (2006.01)     *A61K 8/73* (2006.01)
*A61K 9/46* (2006.01)     *A61K 45/06* (2006.01)
*A61K 47/02* (2006.01)     *A61K 47/12* (2006.01)
*A61K 8/38* (2006.01)     *A61Q 19/10* (2006.01)
*A61K 9/00* (2006.01)     *A61K 9/12* (2006.01)
*A61K 8/04* (2006.01)     *A61K 31/327* (2006.01)
*A61K 47/36* (2006.01)     *A61K 8/19* (2006.01)

(86) Numéro de dépôt international:
**PCT/EP2015/056783**

(87) Numéro de publication internationale:
**WO 2015/144905 (01.10.2015 Gazette 2015/39)**

(54) **MOUSSE CHIMIQUE RINCÉE CONTENANT DU PEROXYDE DE BENZOYLE**

CHEMISCHER BENZOYLPEROXID ENTHALTENDER GESPÜLTER SCHAUM

RINSED CHEMICAL FOAM CONTAINING BENZOYL PEROXIDE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **28.03.2014 US 201461971658 P**

(43) Date de publication de la demande:
**01.02.2017 Bulletin 2017/05**

(73) Titulaire: **Galderma Research & Development
06410 Biot (FR)**

(72) Inventeurs:
• **BUGE, Jean-Christophe**
**06200 Nice (FR)**
• **NADAU-FOURCADE, Karine**
**06270 Villeneuve Loubet (FR)**

(74) Mandataire: **Casalonga
Casalonga & Partners
Bayerstraße 71/73
80335 München (DE)**

(56) Documents cités:
**WO-A1-00/27356     WO-A1-2005/058272**

• **Anonymous: "Benzefoam Ultra", Drug
Information Online - Drugs.com , 3 avril 2011
(2011-04-03), XP002739766, Extrait de l'Internet:
URL:http://dailymed.nlm.nih.gov/dailymed/a
rchives/fdaDrugInfo.cfm?archiveid=51198
[extrait le 2015-05-07]**

**Description**

La présente invention a pour objet un produit topique nettoyant rincé sous forme d'une mousse pour le traitement cosmétique et/ou pharmaceutique des peaux acnéiques comprenant du peroxyde de benzoyle sous forme dispersée.

Malgré les nouvelles générations de tensio-actifs moussants, les produits d'hygiène et tout particulièrement les produits nettoyants restent irritants et peuvent entrainer une tolérance médiocre du produit. Cependant, l'utilisateur associe le volume et la quantité de mousse au pouvoir nettoyant et à l'efficacité de celui-ci. Il existe donc un besoin de mettre au point de nouvelles formes galéniques moussantes contenant peu ou pas de tensio-actifs moussants qui soient à la fois efficaces et respectent les attentes de l'utilisateur, tout en améliorant la tolérance de ce genre de produit.

Depuis de nombreuse année, le BPO ou peroxyde de benzoyle de formule brute $C_{14}H_{10}O_4$ est un agent thérapeutique préconisé dans le traitement de l'acné. Tout d'abord, l'efficacité du peroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir au peroxyde de benzoyle une efficacité optimale, il est important de prévenir sa décomposition avant utilisation, c'est à dire durant le stockage.

Or le peroxyde de benzoyle est un composé chimique instable, ce qui rend difficile sa formulation dans des produits finis. Le BPO présente en outre une réactivité croisée avec les autres ingrédients usuellement employés dans les formulations topiques ce qui, soit en limite l'utilisation, soit nécessite de trouver d'autres formes galéniques. On peut citer à titre d'exemple la réactivité du BPO en présence de tensioactifs dans la composition.

De plus, le BPO est particulièrement reconnu pour être difficilement toléré par les consommateurs suivant un traitement contre l'acné. Cette tolérance vis-à-vis du BPO est très variable en fonction de la formule lui servant de véhicule.

Ainsi, Il existe donc le besoin de disposer de nouvelles formes galéniques de produit nettoyant moussant dans lesquelles le peroxyde de benzoyle est stable, bien toléré, efficace et agréable à appliquer.

En effet, de manière générale, les compositions moussantes contiennent une quantité importante de tensio-actifs moussants. Cette forte teneur génère de l'irritation cutanée. La présente invention vise à proposer une composition qui soit particulièrement bien tolérée, comme le montrent les exemples illustrant une des méthodes d'évaluation de la tolérance présentés ci-après.

Il existe différentes méthodes d'évaluation de la tolérance d'un produit pharmaceutique ou cosmétique à usage cutané parmi lesquels on peut citer le test in vivo « in used » or « human patch test », mais aussi le test in vitro tel que le test de mesure de l'irritation sur Epiderme humain reconstruit (Reconstructed Human Epidermis ou RHE) décrit dans le protocole TG439 OCDE. Cette dernière méthode est détaillée dans l'exemple 3.

Il existe actuellement des mousses ou compositions moussantes nettoyantes sur le marché. Toutefois, elles présentent toutes un certain nombre d'inconvénients.

En effet, il existe quatre types de mousses ou compositions moussantes nettoyantes:

- Des aérosols dans lesquels la mousse est générée par un gaz propulseur mais avec l'inconvénient d'être des aérosols présentant les risques bien connus de ceux-ci (pollution, risques respiratoires notamment).
- Des formules riches en tensioactifs moussants. Ces formules présentent l'inconvénient d'être dans le meilleur des cas légèrement irritantes, le plus souvent elles sont irritantes. De plus, les actifs sensibles à la présence de tensioactifs moussants en quantité assez importante ne peuvent pas être envisagés dans ce type de composition.
- Des crèmes foisonnées rinçables dans lesquelles des bulles d'air sont introduites dans le produit grâce à un procédé de fabrication particulier. Ce procédé de fabrication présente l'inconvénient d'être très contraignant au niveau industriel et nécessite un investissement lourd au niveau du matériel industriel de conditionnement.
- Des formules moussantes peu riches en tensioactifs moussants mais conditionnées dans un packaging/emballage muni d'un système mécanique générateur de mousse (pompe avec grille de type pulvorex). Ce type de formulation présente l'inconvénient de ne pas être compatible avec l'utilisation d'actifs sous forme dispersée.

Ainsi, il subsiste donc le besoin de mettre au point une composition cosmétique ou pharmaceutique dont la forme galénique est différente des formes galéniques connues afin de pallier leurs inconvénients et de permettre ainsi l'utilisation de BPO sous forme dispersée dans des compositions moussantes, nettoyantes, rinçables et bien tolérées destinées à une application topique chez l'être humain.

Le but de la présente invention est donc de proposer une telle composition répondant à ces besoins.

La demanderesse a ainsi mis au point une nouvelle composition cosmétique et/ou pharmaceutique, destinée à une application topique rincée qui se présente sous la forme d'une mousse avec peu ou sans tensioactif moussant, c'est-à-dire avec une teneur en tensioactif moussant qui soit avantageusement inférieure ou égale à 1% en poids de matière active. On entend par tensioactif moussant des tensioactifs qui produisent une mousse volumineuse, stable et onctueuse lorsqu'ils sont mélangés à l'eau selon les tests bien connus de l'homme du métier.

Constituent notamment des tensioactifs moussants : les tensioactifs anioniques, les tensioactifs cationiques, les tensioactifs amphotères et les tensioactifs non ioniques de la famille des alkylpolyglucosides et des glucamides.

La forme galénique selon l'invention présente l'avantage de garantir une bonne stabilité du BPO. De plus, cette formulation conduit avantageusement à l'obtention d'une mousse douce, parfaitement tolérée et non irritante, qui permet le traitement et le nettoyage des peaux acnéiques tout en palliant les problèmes de tolérance et en satisfaisant le client en matière de qualité de mousse.

Enfin, de manière avantageuse, cette forme galénique ne nécessite pas pour sa mise en oeuvre l'utilisation de gaz propulseurs ou d'aérosols. Ainsi les mousses dites aérosol ou spray sont exclues du champ de l'invention. De même, les compositions moussantes rinçables de l'art antérieur de type compositions moussantes classiques riches en tensioactifs moussants et/ou les formules moussantes à teneur plus réduites en tensioactifs mais nécessitant un système mécanique générateur de mousse (type pulvorex) sont également exclues de l'invention. Il en va de même des compositions moussantes faisant intervenir un procédé de foisonnage.

La présente invention a aussi pour objet une composition pour son utilisation comme médicament par application topique sur la peau. Selon l'invention, après son application la composition selon l'invention est éliminée par rinçage. La présente invention sera décrite plus en détail dans la description et les exemples ci-après, et au vu des Figures annexées à la présente demande.

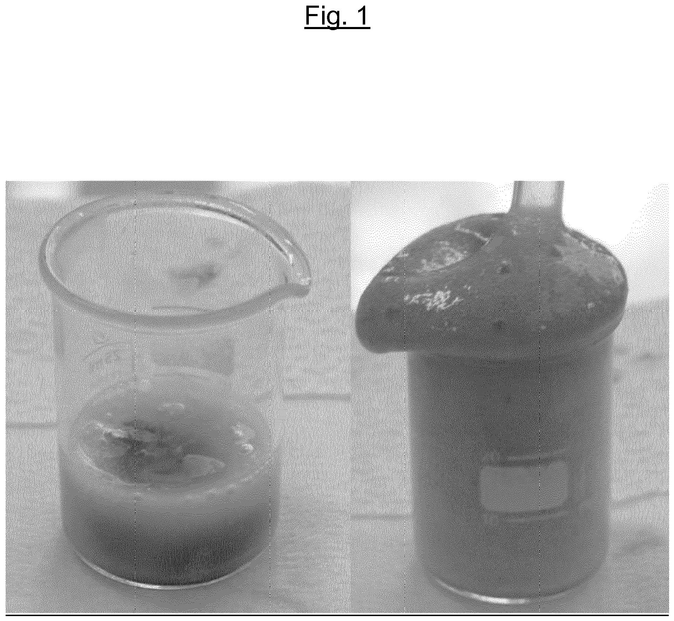

La Figure 1 montre des photographies d'une première composition conforme à l'invention obtenue par mélange des deux compositions intermédiaires A5 et B1 décrites dans les exemples, immédiatement après mélange de celles-ci puis lorsque la réaction entre ces deux compositions est complète (volume de mousse maximal).

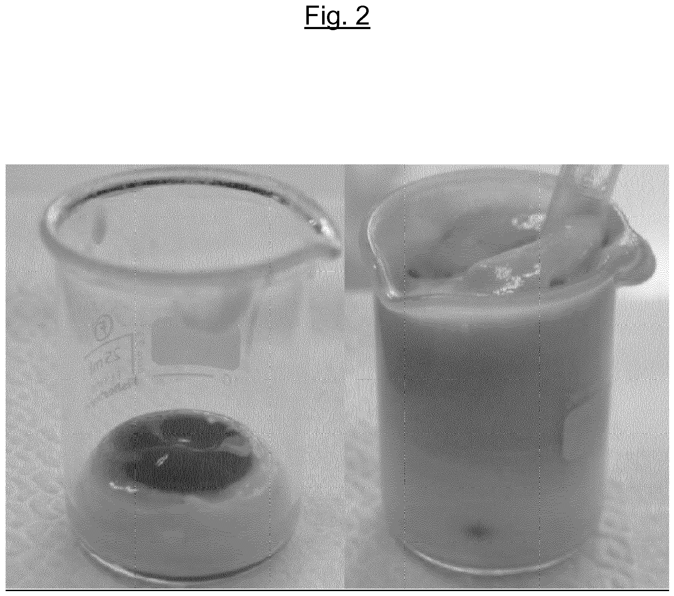

La Figure 2 montre des photographies d'une seconde composition conforme à l'invention obtenue par mélange des deux compositions intermédiaires A5 et B2 décrites dans les exemples, immédiatement après mélange de celles-ci puis lorsque la réaction entre ces deux compositions est complète (volume de mousse maximal).

Cette composition est capable de prendre la forme d'une mousse par le seul fait de sa composition et peut ainsi être également définie comme une composition auto-moussante pour application topique.

La présente invention a, en conséquence, pour premier objet une composition contenant du peroxyde de benzoyle destinée à une application topique se présentant sous la forme d'une mousse, avantageusement de consistance semi-solide, avec peu ou sans tensioactif moussant (teneur inférieure ou égale à 1% en poids, par rapport au poids de la composition totale) comprenant un milieu cosmétiquement ou pharmaceutiquement compatible avec une application topique rincée notamment sur la peau.

Par composition se présentant sous la forme d'une mousse, (également dénommée ci-après composition auto-moussante), il est entendu une composition de consistance semi-solide ayant une forme aérée assimilable à une mousse.

La composition auto-moussante comprend au moins deux compositions ou formules intermédiaires dans des proportions variables et notamment les ingrédients ci-après :

- au moins une composition ou formule intermédiaire A comprenant un agent activateur de l'agent générateur de gaz choisi parmi un acide, un sel de polyacide partiellement salifié , une solution tampon d'acide faible et de sa base conjuguée, et les mélanges de ces composés, et
- au moins une composition ou formule intermédiaire B comprenant un agent générateur de gaz choisi parmi le bicarbonate de sodium, le bicarbonate de potassium, le carbonate de sodium, le carbonate de potassium et leurs mélanges, et
- du peroxyde de benzoyle contenu dans l'une au moins desdites formules intermédiaires A et B.

De préférence, le peroxyde de benzoyle est contenu dans la composition intermédiaire A.

La composition selon l'invention est automoussante, c'est-à-dire qu'elle mousse par simple mélange des compositions intermédiaires A et B.

En fonction des agents présents dans la composition intermédiaire (ou formule intermédiaire), ainsi que de leurs proportions dans ladite composition, celle-ci peut se présenter sous toutes les formes galéniques ou prendre toutes les textures connues utilisables en cosmétique et/ou en pharmacie, pour une application topique.

De manière préférée, chaque composition intermédiaire (ou formule intermédiaire) selon l'invention peut donc se présenter par exemple sous forme de gel, émulsion (crème, crème sans tensioactif, lotion, lait ou crème fluide), sérum, solution, suspension, et préférentiellement sous la forme d'émulsion (crème, crème sans tensioactif, lotion, lait ou crème fluide) ou de gel. L'ensemble de ces formulations entrent dans la définition de la composition mousseuse dans le cadre de l'invention.

Selon l'invention, chaque composition (ou formule) intermédiaire peut présenter une viscosité (mesurée à 25°C et à pression atmosphérique) comprise entre 1cP et 500000 cP, avantageusement entre 500 cP et 350000 cP, mesurée avec une méthode classique de type brookfield RV DV II : Aiguille 6 vit 2.

Selon l'invention le gaz généré par l'agent générateur de gaz peut être tout gaz physiologiquement compatible et permettant l'obtention d'une mousse, comme par exemple le dioxyde de carbone ($CO_2$) ou l'oxygène ($O_2$).

Selon l'invention, la concentration en gaz pouvant varier, la quantité de bulles dans la composition peut varier et ainsi donner une composition pouvant aller de peu aérée à très fortement aérée.

Selon l'invention, on entend par agent activateur de l'agent générateur de gaz un ingrédient qui, par réaction chimique avec l'agent générateur de gaz, libère un gaz. Préférentiellement, il s'agit d'une réaction acide/base.

Ainsi selon l'invention, la composition auto-moussante peut se présenter préférentiellement sous toutes les formes allant de aérée à une mousse très expansée.

La composition selon l'invention est adaptée à une application topique et peut comprendre en outre un milieu physiologiquement acceptable, c'est-à-dire un milieu compatible avec la peau et les phanères. Il s'agit de préférence d'un milieu cosmétiquement ou pharmaceutiquement acceptable.

En outre, la composition peut comprendre tout agent actif susceptible de présenter une activité, éventuellement thérapeutique. Ces agents actifs peuvent être, entre autres, choisis parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

La composition selon l'invention peut comprendre, en plus des actifs cités ci-dessus, des adjuvants cosmétiquement et/ou pharmaceutiquement acceptables, tels que des dispersants, des solubilisants, des stabilisants, des conservateurs, des phases grasses, des corps gras, des agents épaississants, des colorants, des parfums, des tensioactifs non moussants, des gélifiants, des complexants, des neutralisants, des agents masqueurs d'odeur, des charges, des séquestrants, des agents réducteurs, des plastifiants, des adoucissants, des agents hydratants, des pigments, des argiles, des charges minérales, des colloïdes minéraux, des polymères, des protéines, des agents nacrants, des cires, des huiles comme par exemple les paraffines, des acides gras, des esters solides d'alcool gras ou d'acides gras, des gommes, des agents mouillants.

Bien entendu, l'homme du métier veillera à choisir ce ou ces éventuels adjuvants additionnels et/ou leur quantité de manière telle que les propriétés de (ou des) actif(s) pouvant être ajoutés à la composition selon l'invention ne soient pas, ou substantiellement pas, altérées par l'adjonction envisagée.

Des colorants tels que le FD&C Blue 1 peuvent également être présents dans l'une au moins des compositions intermédiaires. De tels colorants présentent l'avantage de colorer un des intermédiaires de formulation. Cette coloration permet le contrôle du bon mélange des deux intermédiaires de formulation et de mettre en valeur la formation de la mousse.

Selon l'invention, la composition auto-moussante (c'est-à-dire prête à être appliquée) peut avoir un pH compris entre 2 et 8, préférentiellement entre 3 et 7.

Dans la mesure où la ou les composition(s) (ou formule(s)) intermédiaire(s) nécessite(nt) un stockage en au moins 2 compartiments pour des raisons de stabilité des ingrédients, la présente invention se rapporte soit à un unique contenant compartimenté (chaque compartiment accueillant une formule intermédiaire) et de préférence comprenant 2 ou 3 compartiments soit à un kit comprenant chaque formule intermédiaire stockée indépendamment l'une de l'autre et physiquement séparées.

Le mélange intime extemporané (directement sur la peau ou sur tout autre support) des formules intermédiaires permet d'obtenir la composition auto-moussante selon l'invention.

De manière plus spécifique, la composition (ou formule) intermédiaire A peut se présenter sous forme d'une solution, d'une émulsion (lotion, crème, crème sans émulsionnant, lait, crème fluide) ou gel. Cette composition contient l'agent activateur de l'agent générateur de gaz, préférentiellement un acide en quantité suffisante (pouvant se présenter sous forme d'un tampon acide/Base à pH acide) qui peut être à titre d'exemple non limitatif le couple Acide Citrique/Citrate de sodium.

La formule B peut se présenter sous forme d'une solution, d'un gel, d'une émulsion (lotion, crème, crème sans émulsionnant, lait, crème fluide). Cette composition contient en quantité suffisante l' agent générateur de gaz, qui peut être en particulier du bicarbonate de sodium.

Ainsi, l'invention a également pour objet un kit ou un contenant unique à plusieurs compartiments tel que défini précédemment, permettant la préparation extemporanée d'une composition auto-moussante selon l'invention, comprenant de manière séparée au moins deux formules intermédiaires (ou compositions intermédiaires):

- une formule intermédiaire A comprenant au moins l' agent activateur de l'agent générateur de gaz; et
- une formule intermédiaire B comprenant au moins l' agent générateur de gaz ;
- du peroxyde de benzoyle étant contenu dans l'une au moins desdites formules intermédiaires A et B.

De préférence, le peroxyde de benzoyle est contenu dans la composition intermédiaire A.

AGENT ACTIVATEUR DE GAZ :

L'agent activateur de l'agent générateur de gaz (également désigné par « agent activateur de gaz ») est un composé qui réagit avec l'agent générateur de gaz par une réaction chimique (préférentiellement une réaction acido-basique) libérant un gaz.

Il s'agit d'un acide, d'un sel de polyacide partiellement salifié ou bien d'une solution tampon d'acide faible et de sa base conjuguée, ou d'un mélange de tels composés.

Selon l'invention le tampon acide/base dudit acide peut être tout tampon acide /base de l'acide faible comme par exemple un tampon acide citrique/citrate de sodium ou encore un tampon acide tartrique/tartrate de sodium. De préférence, nous citerons les alpha-hydroxyacides qui sont des acides faibles ayant préférentiellement un pKa compris entre 2 et 6 tel que l'acide citrique, l'acide tartrique, l'acide malique, l'acide lactique mais aussi l'acide phosphorique et l'acide pyrophosphorique et leurs sels partiellement salifiés tels que le disodium pyrophosphate ou le sodium dihydrogénophosphate appelé encore phosphate monosodique

Préférentiellement selon l'invention, l'agent activateur de gaz est choisi parmi un tampon acide citrique/citrate de sodium seul, l'acide phosphorique, le phosphate de sodium, le disodium pyrophosphate seuls ou en mélange avec le tampon acide citrique/ citrate de sodium.

Selon un mode de réalisation très préféré, l'agent activateur de gaz est un tampon acide citrique/ citrate de sodium seul ou en mélange avec du phosphate sodique et/ou du disodium pyrophosphate.

Dans des compositions pour peaux sensibles ou pour peaux lésées comme les peaux acnéiques, la teneur en acide citrique/ citrate de sodium est de préférence inférieure ou égale à 2.4% par rapport au poids total de la composition intermédiaire A, de manière à limiter tout risque de picotement. Afin d'améliorer la tolérance et d'éviter la sensation de picotement, de manière préférée, le tampon acide citrique/citrate de sodium est employé en mélange avec le disodium pyrophosphate ou le sodium dihydrogénophosphate.

Selon l'invention, ledit agent activateur de gaz peut être présent dans la formule intermédiaire A en une quantité pouvant aller de 0,001% à 95% en poids par rapport au poids total de la formule intermédiaire A.

AGENT GENERATEUR DE GAZ :

Par agent générateur de gaz, on entend tout agent qui possède la propriété de générer par réaction chimique un gaz. On citera à cet égard tout composé qui, lorsqu'il est mélangé à un acide faible, peut former un gaz par une réaction chimique équivalente à la suivante :

$$NaHCO_3 + RCOOH \rightarrow RCOONa + H_2O + CO_2$$

Selon l'invention, le gaz généré à partir de l'agent générateur de gaz présent dans la composition intermédiaire B est de préférence du gaz carbonique ou dioxyde de carbone ($CO_2$).

Selon l'invention, l'agent générateur de gaz est parmi le bicarbonate de sodium, le bicarbonate de potassium, le carbonate de sodium, le carbonate de potassium et leurs mélanges.

Préférentiellement selon l'invention, la formule intermédiaire B comprend un agent générateur de gaz carbonique qui de manière particulièrement préférée est du bicarbonate de sodium.

Ledit agent générateur de gaz peut être présent dans la formule intermédiaire B en une quantité allant de 1% à10%, préférentiellement de 2% à 8% en poids par rapport au poids de la formule B.

Selon l'invention, la formule intermédiaire A peut présenter un pH acide, avantageusement compris entre 1 et 6, et la formule intermédiaire B peut présenter un pH basique, avantageusement compris entre 7 et 12.

Selon l'invention, l'une (ou les) formule(s) intermédiaires A et B décrites ci-avant, comprend du peroxyde de benzoyle en quantité allant de 0,0001% à 20% de peroxyde de benzoyle, préférentiellement de 0,025% à 10%, encore plus préférentiellement de 2,5% à 5% en poids par rapport au poids de la composition totale.

Dans la présente description, on entend par composition totale ou formule totale la composition du produit sous forme de mousse après le mélange desdites compositions intermédiaires. De façon préférée, le BPO est contenu dans la composition A à pH acide afin d'optimiser sa stabilité.

La formule intermédiaire A peut se présenter sous toutes les formes galéniques compatibles avec la forme galénique désirée pour la composition finale obtenue par mélange de la formule A avec la formule B. Avantageusement la formule A peut-être un gel, une solution, une suspension, une émulsion (crème, crème sans tensioactif, lotion, lait, crème fluide), de préférence un gel garantissant la suspension et la stabilité du BPO. Selon un mode de réalisation particulièrement préféré, la formule intermédiaire A est un gel.

La formule intermédiaire B peut se présenter sous toutes les formes galéniques compatibles avec la forme galénique désirée pour la composition finale obtenue par mélange de la formule B avec la formule A. Avantageusement la formule B peut être un gel, une solution, une suspension, une émulsion (crème, crème sans tensioactif, lotion lait, crème fluide), de préférence un gel, une solution ou une émulsion. Selon un mode de réalisation particulièrement préféré, la formule intermédiaire B est une émulsion, et contient une phase grasse contenant une ou plusieurs huiles telles que décrites ci-après.

Chaque formule du kit ou du contenant à plusieurs compartiments tel que défini précédemment, conforme à l'invention comprend un milieu physiologiquement acceptable, véhiculant le ou les composés, et choisi de telle sorte que les composés soient aptes à réagir l'un avec l'autre pour former une composition auto-moussante lors du mélange d'au moins les formules intermédiaires A et B.

Ainsi, le mélange extemporané d'au moins la formule A et la formule B, crée la composition auto-moussante selon l'invention.

Lors du mélange des deux formules A et B, l'agent générateur de gaz, tel que le bicarbonate de sodium, peut réagir avec l'agent activateur de gaz tel que l'acide, et donner ainsi notamment le sel correspondant à l'acide, de l'eau et le gaz $CO_2$. C'est ce gaz, emprisonné dans les bulles de la composition, qui crée la mousse qui caractérise la composition auto-moussante de l'invention.

Ainsi par le mélange d'au moins la formule intermédiaire A et la formule intermédiaire B, on obtient la composition mousse dite composition totale.

Dans la composition obtenue après mélange d'au moins les formules A et B, il peut bien entendu rester de l'agent activateur de gaz et/ou de l'agent générateur de gaz n'ayant pas réagi.

Avantageusement, le kit ou le contenant unique à plusieurs compartiments selon l'invention peut être conçu de telle sorte que lors de la préparation de la composition selon l'invention, les formules intermédiaires A et B peuvent être mélangées en un rapport en poids A/B allant de 0,5 à 2, préférentiellement de 0,5 à 1,5, plus préférentiellement proche de 1 (c'est-à-dire de 0,9 à 1,1), et encore plus préférentiellement de 1. Cela signifie que le kit peut être conçu pour libérer simultanément des doses (en poids) des compositions intermédiaires A et B pouvant être en un rapport en poids allant de 2 doses de B pour 1 dose de A à 2 doses de A pour 1 dose de B, de préférence de 2 doses de B pour 1 dose de A à 3 doses de A pour 2 doses de B. Selon un mode de réalisation préféré de l'invention, le kit est conçu pour libérer simultanément 1 dose en poids de A et 1 dose en poids de B.

Selon l'invention le kit peut se présenter sous toute forme compatible avec d'une part une conservation séparée des formules intermédiaires A et B et d'autre part la capacité à réaliser extemporanément le mélange de A et de B.

Par exemple les formules intermédiaires A et B peuvent être dans un boitier avec au moins deux compartiments séparés, chacun contenant A ou B.

Selon un autre aspect, le kit peut se présenter sous la forme d'une seringue à au moins deux corps séparés, chacun muni d'un piston, lesdits deux corps contenant A et B, et étant conçus pour libérer simultanément par exercice d'une force sur le piston les doses désirées de A et B.

L'invention concerne également un procédé de préparation d'une composition selon l'invention, caractérisé en ce que pour obtenir la composition auto-moussante, on mélange de façon extemporanée au moins une dose de formule intermédiaire A et une dose de formule intermédiaire B du kit telles que définies plus haut, dans des proportions relatives en poids A/B pouvant aller de 0,5 à 2, préférentiellement de 0,5 à 1,5 et plus préférentiellement de 1.

Afin d'obtenir une mousse (composition finale) optimale, les inventeurs ont cherché expérimentalement les teneurs optimales en agent générateur de gaz (de préférence le bicarbonate de sodium) et en agent activateur de gaz (de préférence l'acide citrique et/ou le disodium pyrophosphate et/ou le sodium dihydrogénophosphate).

Ainsi, il a été déterminé expérimentalement que lorsque l'agent activateur de gaz est l'acide citrique, le ratio pondéral acide citrique/bicarbonate de sodium dans la composition totale est compris entre 0,1 et 2 préférentiellement entre 0,5 à 1 et de façon préférée égal à 0,7.

De la même façon, il a été déterminé que lorsque l'agent activateur de gaz est le disodium pyrophosphate, le ratio pondéral disodium pyrophosphate/bicarbonate de sodium dans la composition totale est compris entre 0,5 et 5 préférentiellement entre 1 et 3 et de façon préférée est égal à 2,4.

De la même façon, il a été déterminé que lorsque l'agent activateur de gaz est le sodium dihydrogénophosphate, le ratio pondéral sodium dihydrogénophosphate mono hydrate/bicarbonate de sodium dans la composition totale est compris entre 0,5 et 5 préférentiellement entre 1 et 3 et de façon préférée est égal à 2.

L'exemplification des ratios bicarbonate de sodium/acide citrique, bicarbonate de sodium/pyrophosphate de sodium, bicarbonate de sodium/sodium hydrogénophosphate est décrit dans l'exemple 4.

De façon surprenante, l'association acide citrique/citrate de sodium, disodium pyrophosphate et un système gélifiant compatible avec la forme galénique a permis d'obtenir une formule aux propriétés physico-chimiques très stables (voir tableau I) et dans laquelle le BPO est particulièrement stable (voir tableau II) ne générant aucune sensation désagréable sur la peau et permettant la libération de gaz et ainsi la création de mousse.

Une composition est considérée stable physiquement lorsque ses caractéristiques organoleptiques, son pH, sa viscosité et l'homogénéité du BPO n'évoluent pas avec le temps dans différentes conditions de températures : température ambiante (RT), 30°C et 40°C. Selon l'invention, la température ambiante correspond à une température allant de 15°C à 25°C.

Tableau I: stabilité physique de la formule intermédiaire A5 (exemple 1) contenant du BPO

| Formule A5 | T0 | | | T3Mois | | T6Mois | |
|---|---|---|---|---|---|---|---|
| pH | 3.54 | 25°C | | 4.0 | | - | |
| | | 30°C | | 4.0 | | - | |
| | | 40°C | | 4.0 | | - | |
| Viscosité cP Brookfield RV DVII | Aig3 vit 5 16000 | 25°C | Aig3 vit 5 | 17600 | Aig3 vit 2.5 | 33400 | |
| | | 30°C | | 18200 | | 37500 | |
| | | 40°C | | 21000 | | 37560 | |

Une composition est considérée stable chimiquement lorsque la teneur en principe actif qu'elle contient n'évolue pas avec le temps dans les différentes conditions de températures (RT, 30°C et 40°C).

Selon l'invention, la composition est considérée stable quand la teneur en BPO (exprimée en poids par rapport au poids de la formule intermédiaire) est incluse dans les spécifications allant de 90% à 110%.

Tableau II : stabilité chimique du BPO dans la formule intermédiaire A5 (exemple 1)

| Formule A5 | T0 | | T1Mois | T2Mois | T3Mois | T6Mois |
|---|---|---|---|---|---|---|
| %p/p BPO (HPLC) | 102 | 25°C | 102.6 | 101.1 | 104.7 | 101.6 |
| | | 30°C | | | 101.8 | 99.0 |
| | | 40°C | 103.4 | 103.8 | 99.3 | 93.6 |

La composition selon l'invention peut comprendre en outre un ou plusieurs agents choisis parmi les agents dispersants, les agents solubilisants, les agents stabilisants, les agents conservateurs, les corps gras, les agents épaississants, les colorants, les parfums, les tensioactifs, les agents gélifiants, les agents complexants, les agents neutralisants, les agents émulsionnants moussants, les agents émulsionnants non moussants, les charges, les agents séquestrants, les agents réducteurs, les masques d'odeur, les agents plastifiants, les agents adoucissants, les agents hydratants, les pigments, les argiles, les charges minérales, les colloïdes minéraux, les polymères, les protéines, les agents nacrants, , les cires, les huiles comme par exemple les paraffines, les silicones, des acides gras, des esters solides d'alcool gras ou d'acides gras, des gommes, les agents mouillants.

Des colorants hydrosolubles tels que le FD&C Blue 1 (de formule brute $C_{37}H_{34}N_2Na_2O_9S_3$) et des colorants liposolubles tel que le Rouge Soudan III ou le Red Nil présentent l'avantage de colorer un des intermédiaires de formulation. Cette coloration permet le contrôle du bon mélange des deux intermédiaires de formulation et de mettre en valeur la formation de la mousse. Cette coloration est notamment présentée dans les exemples et en figure 1.

AGENTS GELIFIANTS DE LA FORMULE INTERMEDIAIRE CONTENANT L'ACTIVATEUR DE GAZ

La composition intermédiaire A contenant au moins l' agent activateur de l'agent générateur de gaz contient de préférence au moins un agent gélifiant et/ou agent de suspension.

Les gels contenant du BPO sont connus pour être très compliqués à stabiliser. La viscosité et le pouvoir suspensif de ces formules sont souvent durs à garantir dans le temps. De plus la formulation A peut contenir des fortes quantités d'acide et d'électrolytes.

A titre d'exemple non limitatif de gélifiants et/ou agents de suspension résistants à la fois au BPO, aux électrolytes et aux pH acides pouvant entrer dans les compositions A selon l'invention, on peut citer les mélanges prêt à l'emploi tels que le Polyacrylate-13 & Polyisobutene & Polysorbate 20 vendus par Seppic sous le nom Sepiplus 400®, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, la famille des amidons modifiés tels que l'amidon de pomme de terre modifié vendu sous le nom de Structure Solanace®, la famille des carraghénanes en particulier réparties sous quatre grandes familles : κ, λ, β, ω tel que les Viscarin® et les Gelcarin® commercialisés par la société IMCD. Ou encore la Polyvinyl Alcohol connue aussi sous l'abréviation PVA revendu par Merck sous le nom POLYVINYL ALCOHOL 40-88®. De façon préférée, le Vegum K® et le Xantural 180® seront utilisés en association.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire A.

AGENTS GELIFIANTS DE LA FORMULE INTERMEDIAIRE CONTENANT LE GENERATEUR DE GAZ

La composition intermédiaire B contenant au moins l'agent générateur de gaz contient de préférence au moins un agent gélifiant et/ou agent de suspension.

A titre d'exemple non limitatif de gélifiants et/ou agents de suspension et/ou gélifiants résistants à la fois aux électrolytes et aux pH basiques pouvant entrer dans les compositions intermédiaires B selon l'invention, on peut citer les polymères d'acide acryliques comme l'Acrylates/C10-30 Alkyl Acrylate Crosspolymer tel que les carbomers dits non sensibles aux électrolytes, vendus sous le nom d'Ultrez 20®, d'Ultrez 10®, de Carbopol 1382® ou de Carbopol ETD2020NF®, AQUA SF1® vendus par la Société Lubrizol, le mélange Ammonium Acrylate / Acrylamide Copolymer & Polyisobutene & Polysorbate 20 vendu par Seppic sous le nom Sepiplus 265®, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M premium® par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, , la famille des amidons modifiés tels que l'amidon de pomme de terre modifié vendu sous le nom de Structure Solanace® ou de Farine de Tapioca connue sous le nom de Naviance Tapioca P® revendu par Akzo Novel ou de la famille des carraghénanes en particulier réparties sous quatre grandes familles : κ, λ, β, ω tel que les Viscarin® et les Gelcarin® commercialisés par la société IMCD.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire B.

AGENTS MOUILLANTS

La composition selon l'invention peut contenir un ou plusieurs agents mouillants. Dans ce cas, ce ou ces agents mouillants sont présents dans la composition intermédiaire qui contient le BPO.

Parmi les agents mouillants qui ont pour rôle de diminuer la tension superficielle et de permettre au BPO d'être incorporé plus facilement dans le formule et principalement au moment de son broyage, on utilise préférentiellement, sans que cette liste soit limitative, un agent mouillant pouvant présenter préférentiellement un HLB de 10 à 14, des composés de la famille des Poloxamers et/ou des glycols et plus particulièrement le Synperonic PE/L44® et/ou le Synperonic PE/L62® et/ou des composés tels que le propylène glycol, le dipropylène glycol, le propylène glycol dipélargonate, le lauroglycol, l'éthoxydiglycol, De préférence, les agents mouillants sont sous forme liquide de manière à s'incorporer aisément dans la composition sans qu'il soit nécessaire de la chauffer.

L'agent mouillant particulièrement préféré est le LUTROL L44® vendu par la société BASF. Il peut être utilisé aux concentrations préférentielles allant de 0,001% à 5 % et, plus préférentiellement, allant de 0.01% à 1% en poids par rapport au poids de la composition totale.

AGENTS HUMECTANTS

Parmi les agents humectants et/ou émollients qui ont pour rôle d'hydrater la peau et de faciliter l'application de la formulation, on utilise optionnellement, sans que cette liste soit limitative, des composés tels qu'un polyol miscible à l'eau à la température ambiante (25°C) notamment choisi parmi les polyols ayant notamment de 2 à 20 atomes de carbones, de préférence ayant de 2 à 10 atomes de carbone, et préférentiellement ayant de 2 à 6 atomes de carbones, tels que le glycérol, les dérivés de glycol tel que le propylène glycol, le butylène glycol, le pentylène glycol, l'hexylène glycol, le dipropylène glycol, le diéthylène glycol et leurs mélanges mais aussi les sucres (à titre d'exemple le glucose, le lactose), les polyethylene glycol (PEG) (à titre d'exemple le Lutrol E400), l'urée, les acides aminés (à titre d'exemple la sérine, la citrulline, l'arginine, l'asparagine, l'alanine).

A titre d'agent humectant et/ou émollient préféré, on peut citer la glycérine et le propylene glycol.

Les agents humectants peuvent être utilisés, seuls ou en association, aux concentrations préférentielles allant de 0,001 % à 30 % et, plus préférentiellement, allant de 0.01% à 10% en poids par rapport au poids de la formule totale.

## AGENTS CHELATANTS

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA). A titre d'agent chélatant préféré, on peut citer l'acide éthylène diamine tétra-acétique (EDTA) vendu notamment sous le nom Titriplex III®. Il peut être utilisé aux concentrations préférentielles allant de 0,001% à 1 % et, plus préférentiellement de 0.05% à 0.1% en poids par rapport au poids de la formule totale.

## ACTIFS COSMETIQUES

La composition selon l'invention peut contenir un ou plusieurs actifs cosmétiques comme par exemple, à titre non limitatif, l'allantoine aux proprietés anti-irritantes, le Zinc gluconate anti-acnéique, le Dipotassium Glycyrrhizate pour ces propriétés anti-inflamatoires ou encore l'alpha Bisabolol cicatrisant.

## CHARGES ET PARTICULES

Des charges et/ou des particules peuvent être utilisées pour stabiliser et booster la mousse. Certaines d'entre elles ont la propriété particulière de se placer à l'interface eau/air et ainsi stabiliser cette interface. Comme charges, on peut citer le talc, les oxydes de métaux tel que l'oxyde de zinc, le dioxyde de titane TiO2 T2000 vendu par la société Merck sous le nom Eusolex® T-2000, les argiles tel que les laponites®, bentones® ou les bentonites® mais aussi les ethers de cellulose tel que le Methocel K100 LV® commercialisé par la société DOW, les silices telles que l'Aerosil® R972 vendue par la société EVONIK ou la SILICE HDK® H13L vendue par WACKER. Elles peuvent être utilisées aux concentrations allant de 0.01% à 10% en poids par rapport au poids de la formule totale.

## LES HUILES DE LA PHASE GRASSE

La composition selon l'invention peut également comprendre une phase grasse.

Cette phase grasse peut comprendre par exemple, des cires, des huiles ou des beurres végétaux, minéraux, animales ou synthétiques, des huiles de silicones, et leurs mélanges.

La phase grasse peut être présente dans l'une et/ou l'autre des compositions intermédiaires A et B. Toutefois, de par l'instabilité du BPO vis-à-vis les molécules lipophiles, lorsque la composition selon l'invention contient une phase grasse, celle-ci est contenue préférentiellement dans la composition intermédiaire B.

La phase grasse de la composition selon l'invention peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive.

Comme huile animale ou leur substitut d'origine végétale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, l'isononyl isononanoate comme le DUB ININ® revendu par les Stearine Dubois, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglyceride tel que Miglyol 812® vendu par la société Univar. Comme polyisobutene hydrogéné, on peut citer les Parleam® vendus par la société Rossow.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning ou encore le Dc 9045 Elastomer Blend® également par la société Dow Corning.

Les phases grasses peuvent selon la forme galénique de chaque intermédiaire de formulation être présentes dans des teneurs allant de 0% à 95% en poids par rapport au poids de chaque formule intermédiaire.

CORPS GRAS NON LIQUIDES

La composition selon l'invention peut également comprendre des corps gras solides tel que des cires naturelles ou synthétiques, des acides gras tels que l'acide stéarique, des alcools gras tels que le Speziol C18 pharma ou le Speziol C16® vendu par la société Cognis, et des agents de texture de type tribehenate, tel que le Compritol 888® vendu par la société Gattefossé ou les huiles de ricin hydrogénées telles que le Cutina HR vendu par la société Cognis ou le glyceryl stearate tel que le GELEOL® vendu par la société Gattefosse. Ces corps gras non liquides peuvent être utilisés seuls ou en mélange de 0% à 30% en poids par rapport au poids de la formule totale. Il a été cependant observé une qualité de mousse exceptionnelle lorsque des alcool gras de formule $CH_3(CH_2)_nOH$ (n est compris entre 11 et 23) sont présents à des teneurs supérieures à 1% en poids par rapport au poids de la formule totale.

EMULSIFIANTS NON IONIQUES

La composition selon l'invention peut également comprendre des émulsifiants non ioniques. Ceux-ci sont en particulier présents dans la ou les formules intermédiaires qui contiennent une phase grasse (émulsions).

A titre d'émulsifiants préférés on peut citer des émulsifiants hydrophiles de type Glyceryl Stearate (and) PEG-100 Stearate vendu sous le nom Arlacel 165FL® par la société Uniquema, des émulsifiants lipophiles de type Glucate SS® et Glucamate SSE®, Polyoxyethylene (21) Stearyl Ether vendu sous le nom Brij721® par la société Uniquema ou encore dans la même famille le Brij S2®, le Brij S20®. La self emulsifying Wax vendue par Croda sous le nom de Polawax NF®. On peut citer également des tensioactifs non ioniques présentant un HLB élevé, les esters de sorbitan tels que le POE(20) sorbitan monooléate, vendu sous le nom de « Tween 80® » (HLB=15) ; le POE(20) sorbitan monostéarate vendu sous le nom de « Tween 60® » (HLB=14.9) ; les éthers d'alcools gras tels que le POE (21) stéaryl ether (HLB= 15.5), ou le ceteareth 20 vendu sous le nom de Eumulgin® B2 PH par Cognis (HLB de 15,5, ou des tensioactifs non ioniques de basse HLB, les esters de sorbitan, tels que le monostéarate de sorbitan (vendu sous le nom de Span 60 par Uniquema), les esters de glycérol tels que le monostéarate de glycérol (Cutina GMS de chez Cognis), les esters de saccharose de bas HLB comme le distéarate de saccharose. Dans un autre mode selon l'invention les tensioactifs utilisables sont les esters de polyglycerol. Il s'agit d'esters d'acides gras polyglycerinés obtenus par condensation de glycerine. Des émulsionnants glycolipidique tel que le Montanov 202® vendu par la société SEPPIC. Certains émulsionnants peuvent être vendus sous forme de mélange tel que les Emulium Kappa® et Emulium Delta® vendu par Gattefosse. Ces Tensio actifs peuvent être utilisés seuls ou en association de façon à ce que le HLB du système soit supérieur à 12 et préférentiellement supérieur à 15.

De tels émulsifiants peuvent être utilisés entre 0.01% et 30 % en poids par rapport au poids de la composition totale, préférentiellement entre 0.1% et 15%, et plus préférentiellement entre 0.5% et 7 %.

AGENT CONSERVATEUR DE LA COMPOSITION B:

On peut citer à titre d'exemple de conservateurs, le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, le benzoate de sodium ou leurs mélanges.

Au titre de système conservateur préféré on peut citer l'association phenoxyethanol et pentylene glycol.

AGENTS NETTOYANTS DE LA COMPOSITION B:

Dans un mode particulier, la composition B contenant l'agent générateur de gaz est une crème sans tensioactif moussant et contient des agents nettoyants non moussants qui à eux seuls ne sont pas capables de générer de la mousse tels que le Disodium PEG-12 Dimethicone Sulfosuccinate vendu par Rodhia sous le nom de Mackanate® ultra Si, le Sodium Cocoamphoacetate ou Disodium Cocoamphodiacetate vendu par Evonik sous le nom Rewoteric AMC® et Rewoteric AM2CNM®, des esters de sucre comme ceux vendus par Sisterna sous le nom PS750-C® ou SP70-C®. Les quantités de ces agents nettoyants sont comprises entre 0 et 1% en poids par rapport au poids total de la formule B.

TENSIOACTIFS MOUSSANTS DE LA COMPOSITION B

Dans un autre mode particulier, la composition B contenant l'agent générateur de gaz est un gel ou une crème et contient une petite quantité de tensioactifs moussants.

La demanderesse a su sélectionner un tensioactif moussant stable dans le temps en présence d'une forte concentration en bicarbonate de sodium. Les tensio-actifs suivants ont été testés dans le mélange : Sodium C14-C16 Olefin Sulfonate revendu par Clariant sous le nom Hostapur® OSB ou bien Hostapur® OS Liq mais aussi le Nans®a LSS 495-H revendu par Hunstman, Rodacal LSS 80 RPB de Rodia, BIO-Terge® AS-90 beads de chez Stepan , le Sodium Cocoyl Glycinate revendu par Clariant sous le nom Hostapon® SG, Sodium Cocoyl Isethionate revendu par Clariant sous le nom Hostapon® SCI85 G, Sodium Lauroyl Methyl Isethionate revendu par Innospec sous le nom Iselux® LG, Decyl Glucoside revendu par Cognis sous le nom Plantacare® 2000 Up, Zinc Coceth Sulfate revendu par Zschimmer & Schwarz sous le nom Zetesol® Zn, Glyceryl Monolaurate revendu par Rossow sous le nom POEM® DL 100, Disodium PEG-5 Laurylcitrate Sulfosuccinate revendu par Evonik sous le nom Rewopol SB C55®.

Parmi les tensioactifs moussants incorporés dans l'intermédiaire de formulation B, le seul qui ait gardé ses propriétés moussantes et ses propriétés organoleptiques (tel que la couleur ou l'odeur) est le Sodium C14-16 Olefin Sulfonate.

Les quantités de ce tensioactif moussant peuvent être comprises entre 0% et 1% en poids par rapport au poids de la formule totale.

Les exemples suivants illustrent l'invention sans en restreindre la portée.

EXEMPLES

EXEMPLE 1 : exemples de formules

Exemples de Formules A : Compositions intermédiaires A contenant l'agent activateur de l'agent générateur de gaz: Les formules intermédiaires A ont été préparées suivant le procédé suivant :

Etape 1 : A une température supérieure à 60°C, Ajouter sous agitation les gélifiants puis le ou les agents activateurs du générateur de gaz à la phase d'eau principale.

Etape 2 : Parallèlement réaliser la phase de broyage contenant le BPO, l'eau, le Propylène glycol et le Poloxamer 124 sous fort cisaillement.

Etape 3 : A une température inférieure à 30°C mélanges les compositions résultant des étapes 1 et 2.

Etape 4 : Ajouter les additifs tels que le colorant Blue 1 pur ou en solution.

Dans les exemples de formules ci-dessous, les quantités sont exprimées en pourcentage par rapport au poids de la formule intermédiaire et non par rapport au poids de la formule totale (on entend par formule totale, le mélange des deux formules intermédiaires).

Exemple A1

| Ingrédients | INCI Name | % |
|---|---|---|
| EAU | WATER | QSP 100 |
| TITRIPLEX III® | DISODIUM EDTA | 0.1 |
| VEEGUM K® | MAGNESIUM ALUMINIUM SILICATE | 2 |
| XANTURAL 180® | XANTHAN GUM | 0.7 |
| BLANOSE CMC 7H4XF PHARM® | CELLULOSE GUM | 0.4 |
| ACIDE CITRIQUE MONOHYDRATE | CITRIC ACID | 3 |
| SODIUM CITRATE TRISODIQUE | SODIUM CITRATE | 0.8 |
| LUTROL L44® | POLOXAMER 124 | 0.2 |
| PROPYLENE GLYCOL | PROPYLENE GLYCOL | 4 |
| BPO | BENZOYL PEROXIDE | 5.2 |

Exemple A2

| Ingrédients | INCI Name | % |
|---|---|---|
| EAU | WATER | QSP 100 |
| TITRIPLEX III®® | DISODIUM EDTA | 0.1 |

(suite)

| Ingrédients | INCI Name | % |
|---|---|---|
| AMIGEL GRANULE | SCLEROTIUM GUM | 1 |
| XANTURAL 180® | XANTHAN GUM | 0.3 |
| BLANOSE CMC 7H4XF® PHARM | CELLULOSE GUM | 0.4 |
| ACIDE CITRIQUE H20 | CITRIC ACID | 3 |
| SODIUM CITRATE 3H20 | SODIUM CITRATE | 0.8 |
| LUTROL L44® | POLOXAMER 124 | 0.2 |
| PROPYLENE GLYCOL | PROPYLENE GLYCOL | 4 |
| BPO | BENZOYL PEROXIDE | 5.2 |

Exemple A3

| Ingrédients | INCI Name | % |
|---|---|---|
| EAU | WATER | QSP 100 |
| SATIAXANE UCX 911® | XANTHAN GUM | 0,6 |
| VEEGUM K® | MAGNESIUM ALUMINIUM SILICATE | 2.5 |
| ACIDE CITRIQUE MONOHYDRATE | CITRIC ACID | 3,6 |
| SODIUM CITRATE TRISODIQUE | SODIUM CITRATE | 2.6 |
| LUTROL L44® | POLOXAMER 124 | 0,2 |
| BPO | BENZOYL PEROXIDE | 10% |
| PROPYLENE GLYCOL | PROPYLENE GLYCOL | 4,0 |

Exemple A4

| Ingrédients | INCI Name | % |
|---|---|---|
| EAU | WATER | QSP 100 |
| SATIAXANE UCX 911® | XANTHAN GUM | 0,7 |
| VEEGUM K® | MAGNESIUM ALUMINIUM SILICATE | 2.5 |
| FD&C BLUE 1 | FD&C BLUE 1 | 0.0003 |
| DISODIUM PYROPHOSPHATE | DISODIUM PYROPHOSPHATE | 12 |
| LUTROL L44® | POLOXAMER 124 | 0,2 |
| BPO | BENZOYL PEROXIDE | 5.2% |
| PROPYLENE GLYCOL | PROPYLENE GLYCOL | 4,0 |

Exemple A5

| Ingrédients | INCI Name | % |
|---|---|---|
| EAU | WATER | QSP 100 |
| TITRIPLEX III®® | DISODIUM EDTA | 0.1 |
| SATIAXANE UCX 911 | XANTHAN GUM | 0,7 |
| VEEGUM K® | MAGNESIUM ALUMINIUM SILICATE | 2.5 |

(suite)

| Ingrédients | INCI Name | % |
|---|---|---|
| DISODIUM PYROPHOSPHATE | DISODIUM PYROPHOSPHATE | 6 |
| ACIDE CITRIQUE MONOHYDRATE | CITRIC ACID | 1.8 |
| SODIUM CITRATE TRISODIQUE | SODIUM CITRATE | 1.3 |
| LUTROL L44® | POLOXAMER 124 | 0,2 |
| BPO | BENZOYL PEROXIDE | 5.2% |
| PROPYLENE GLYCOL | PROPYLENE GLYCOL | 4,0 |

Exemple A6

| Ingrédients | INCI Name | % |
|---|---|---|
| EAU | WATER | QSP 100 |
| TITRIPLEX III® | DISODIUM EDTA | 0.1 |
| SATIAXANE UCX 911® | XANTHAN GUM | 0,7 |
| VEEGUM K® | MAGNESIUM ALUMINIUM SILICATE | 2.5 |
| DISODIUM PYROPHOSPHATE | DISODIUM PYROPHOSPHATE | 8.4 |
| ACIDE CITRIQUE MONOHYDRATE | CITRIC ACID | 1.4 |
| SODIUM CITRATE TRISODIQUE | SODIUM CITRATE | 1 |
| LUTROL L44® | POLOXAMER 124 | 0,2 |
| BPO | BENZOYL PEROXIDE | 5.2% |
| PROPYLENE GLYCOL | PROPYLENE GLYCOL | 4,0 |
| FD&C BLUE 1 | FD&C BLUE 1 | 0.0005 |

Exemple A7

| Ingrédients | INCI Name | % |
|---|---|---|
| EAU | WATER | QSP 100 |
| TITRIPLEX III® | DISODIUM EDTA | 0.1 |
| SATIAXANE UCX 911® | XANTHAN GUM | 0,7 |
| VEEGUM K® | MAGNESIUM ALUMINIUM SILICATE | 2.5 |
| PROBENZ SP® | SODIUM BENZOATE | 0.2 |
| POLYVINYL ALCOHOL 40-88® | POLYVINYL ALCOHOL | 2 |
| DISODIUM PYROPHOSPHATE | DISODIUM PYROPHOSPHATE | 7.2 |
| PHENOXETHOL® | PHENOXYETHANOL | 0.5 |
| ACIDE CITRIQUE MONOHYDRATE | CITRIC ACID | 1.4 |
| SODIUM CITRATE TRISODIQUE | SODIUM CITRATE | 1 |
| LUTROL L44® | POLOXAMER 124 | 0,2 |
| BPO | BENZOYL PEROXIDE | 5.2% |
| PROPYLENE GLYCOL | PROPYLENE GLYCOL | 4,0 |
| FD&C BLUE 1 | FD&C BLUE 1 | 0.0009 |

Exemple A8

| Ingrédients | INCI Name | % |
|---|---|---|
| EAU | WATER | QSP 100 |
| TITRIPLEX III® | DISODIUM EDTA | 0.1 |
| SATIAXANE UCX 911® | XANTHAN GUM | 0,7 |
| VEEGUM K® | MAGNESIUM ALUMINIUM SILICATE | 2.5 |
| PROBENZ SP® | SODIUM BENZOATE | 0.2 |
| SODIUM DIHYDROGENOPHOPHATE | SODIUM PHOPHATE | 6.2 |
| PHENOXETHOL® | PHENOXYETHANOL | 0.5 |
| ACIDE CITRIQUE MONOHYDRATE | CITRIC ACID | 1.5 |
| SODIUM CITRATE TRISODIQUE | SODIUM CITRATE | 0.5 |
| LUTROL L44® | POLOXAMER 124 | 0,2 |
| BPO | BENZOYL PEROXIDE | 5.2% |
| PROPYLENE GLYCOL | PROPYLENE GLYCOL | 4,0 |

Exemples de Formules B : Compositions intermédiaires B contenant l'agent générateur de gaz.

Les formules intermédiaires B ont été préparées suivant le procédé suivant :

Etape 1' : A une température supérieure à 60°C, Ajouter sous agitation les gélifiants à la phase d'eau principale.

Etape 2' : Ajouter les agents nettoyants ou moussants ainsi que les additifs tels que les conservateurs à une température adaptée.

Etape 3' : Neutraliser le mélange.

Etape 4' : A une température inférieure à 40°C ajouter le bicarbonate de sodium.

Dans un mode particulier, une phase grasse (contenant les huiles, les cires, et les tensio-actifs) peut être chauffée à une température supérieure à 60°C et incorporée à la phase principale après l'étape 1'.

Formule B1

| Ingrédients | INCI Name | % |
|---|---|---|
| EAU | WATER | QSP100 |
| VEEGUM K® | MAGNESIUM ALUMINIUM SILICATE | 2.5 |
| SATIAXANE UCX 911® | XANTHAN GUM | 0.5 |
| TITRIPLEX III® | DISODIUM EDTA | 0.1 |
| PLANTACTIV PGL® | DIPOTASSIUM GLYCYRRHIZATE | 0.5 |
| ZINC GLUCONATE® | ZINC GLUCONATE | 0.4 |
| HOSTAPUR OSB® | (C14-16) SODIUM OLEFIN SULFONATE | 2 |
| NAOH 10% EN SOL AQ. | SODIUM HYDROXYDE | 1 |
| BICARBONATE DE SODIUM | SODIUM HYDROGENO CARBONATE | 5 |
| PHENOXYETHANOL | PHENOXYETHANOL | 1 |
| HYDROLITE 5 | PENTYLENE GLYCOL | 5 |

Formule B2

| Ingrédients | INCI Name | % |
|---|---|---|
| EAU | WATER | QSP100 |
| VEEGUM K® | MAGNESIUM ALUMINIUM SILICATE | 2.5 |
| SATIAXANE UCX 911® | XANTHAN GUM | 0.6 |
| TITRIPLEX III® | DISODIUM EDTA | 0.1 |
| PLANTACTIV PGL® | DIPOTASSIUM GLYCYRRHIZATE | 0.5 |
| ZINC GLUCONATE | ZINC GLUCONATE | 0.4 |
| EUMULGIN B2® | CETEARETH-20 | 3 |
| MACKAMATE ULTRA-SI® | DISODIUM PEG-12 DIMETHICONE SULFOSUCCINATE | 2 |
| PROBENZ® | POTASSIUM SORBATE | 0.5 |
| PARLEAM® | HYDROGENATED POLYISOBUTENE | 6 |
| SPEZIOL C16-C18® | CETOSTEARYL ALCOHOL | 7 |
| NAOH 10% EN SOL AQ. | SODIUM HYDROXYDE | 1 |
| BICARBONATE DE SODIUM | SODIUM HYDROGENO CARBONATE | 5 |
| PHENOXYETHANOL | PHENOXYETHANOL | 1 |

Formule B3

| Ingrédients | INCI Name | % |
|---|---|---|
| EAU | WATER | QSP100 |
| VEEGUM K® | MAGNESIUM ALUMINIUM SILICATE | 2.5 |
| SATIAXANE UCX 911® | XANTHAN GUM | 0.6 |
| TITRIPLEX III® | DISODIUM EDTA | 0.1 |
| PLANTACTIV PGL® | DIPOTASSIUM GLYCYRRHIZATE | 0.5 |
| ZINC GLUCONATE | ZINC GLUCONATE | 0.4 |
| EUMULGIN B2® | CETEARETH-20 | 3 |
| MACKAMATE ULTRA-SI® | DISODIUM PEG-12 DIMETHICONE SULFOSUCCINATE | 2 |
| PROBENZ® | POTASSIUM SORBATE | 0.5 |
| PARLEAM® | HYDROGENATED POLYISOBUTENE | 6 |
| SPEZIOL C16-C18® | CETOSTEARYL ALCOHOL | 7 |
| NAOH 10% EN SOL AQ. | SODIUM HYDROXYDE | 1 |
| CARBONATE DE SODIUM | SODIUM CARBONATE | 5 |
| PHENOXYETHANOL | PHENOXYETHANOL | 1 |

Le tableau ci-dessous représente les mélanges dans un ratio pondéral 1:1 des compositions intermédiaires A et B décrites ci-avant. Une croix à l'intersection de deux intermédiaires de formulation indique que le mélange est possible et génère une mousse aux propriétés recherchées.

| Formule B / Formule A | B1 | B2 | B3 |
|---|---|---|---|
| A1 | X | X | X |
| A2 | X | X | X |
| A3 | X | X | X |
| A4 | X | X | X |
| A5 | X | X | X |
| A6 | X | X | X |
| A7 | X | X | X |

EXEMPLE 2 : mesures de densité

A partir des exemples de formules décrites dans l'exemple 1, des mesures de densité de mousses ont été réalisées au moment de la mise en contact des 2 formules intermédiaires A et B (T0) puis lorsque la réaction chimique générée par la mise en contact des deux compositions est terminée :

a)

Densité formule A5 = 1.108
Densité formule B1 = 1.052
Mousse A5/B1 (50/50)= 0.251

La mesure de la densité de la mousse montre que le volume a été augmenté d'un facteur 5 et confirmé par les photos en Figure 1. La photo de gauche représente le moment du mélange (T0) et la photo de droite représente la mousse obtenue lorsque la réaction chimique acide/ base est complète.

b)

Densité formule A5 = 1.108
Densité formule B2 = 1.012
Mousse A5/B2 (50/50)= 0.340

La mesure de la densité de la mousse montre que le volume a été augmenté d'un facteur 3 et confirmé par les photos en Figure 2. La photo de gauche représente le moment du mélange (T0) et la photo de droite représente la mousse obtenue lorsque la réaction chimique acide/ base est complète.

EXEMPLE 3 : Etude comparative de mesure de l'irritation

Protocole d'étude.

L'étude est réalisée selon le protocole TG439 OCDE en vigueur pour le temps d'application court (temps de contact RHE/produit 15min). Ce protocole est adapté pour un temps d'application long (temps de contact RHE/produit 18h).
L'objectif de cette étude est d'évaluer la tolérance des supports des formules complètes et intermédiaire sur épidermes humains reconstruits (RHE, modèle Episkin) au travers de :

- l'évaluation de la réduction du MTT (viabilité cellulaire)
- la mesure de la libération d'IL-1 alpha (marqueur d'irritation)

Les formules testées sont :

- Une composition intermédiaire de formulation acide : exemple A7 placebo (c'est à dire, ne contenant pas de BPO) non colorée (c'est-à-dire, ne contenant pas de colorant)

- Une composition intermédiaire de formulation basique: exemple B1

- Une composition intermédiaire de formulation basique: exemple B2

- La formule complète 1 composée du mélange : A7 placebo + B1 (dans un ratio 50/50 en poids)

- La formule complète 2 composée du mélange : A7 placebo + B2 (dans un ratio 50/50 en poids)

- Une référence commerciale sous forme gel nettoyant

Résultats de l'étude :

| Mélange testé | Exposition courte | Exposition longue | Conclusion |
|---|---|---|---|
| | Viabilité (%) | Viabilité (%) | Potentiel irritant |
| B1 | 92.5 | 59.6 | Non irritant |
| A7 placebo | 86.0 | 84.5 | Non irritant |
| Formule complète 1 | 93.2 | 86.8 | Non irritant |
| Formule complète 2 | 95.8 | 83.4 | Non irritant |
| Ref commerciale | 76.6 | 6.7 | Potentiellement irritant |

| Test item | Exposition courte | Exposition longue | Conclusion |
|---|---|---|---|
| | IL-1a vs contrôle | IL-1a vs contrôle | Potentiel irritant |
| B1 | 5.5 | 30.6 | Non irritant |
| A7 placebo | 2.2 | 2.3 | Non irritant |
| Formule complète 1 | 4.5 | 11.6 | Non irritant |
| Formule complète 2 | 1.9 | 3.1 | Non irritant |
| Ref commerciale | 6.2 | 113.9 | Potentiellement irritant |

Les mesures du MTT selon le protocole OCDE en vigueur indiquent que les « Formules complètes » rincées testées sont non irritantes alors que la « Ref commerciale » est potentiellement irritante.
De plus, le dosage d'IL-1a après application à des temps d'exposition longs et courts des formules complètes montre un taux de marqueurs d'irritation beaucoup plus bas sur les formules mousses qu'après application de la référence commerciale.

EXEMPLE 4 :

Il a été établi de façon empirique la teneur idéale en acide citrique, pyrophosphate de sodium et sodium dihydrogénophosphate monohydrate pour réagir avec 5% de bicarbonate de sodium. Les valeurs sont exprimées en pourcentage poids/poids par rapport au poids de chacune des deux formules intermédiaires

| | Ratio1 | Ratio2 | Ratio3 |
|---|---|---|---|
| Bicarbonate de Sodium | 5% | 5% | 5% |

(suite)

|  | Ratio1 | Ratio2 | Ratio3 |
|---|---|---|---|
| Acide citrique | 3.5% | - | - |
| Disodium pyrophosphate | - | 12 | - |
| Sodium Dihydrogénophosphate monohydrate |  | - | 7.2% |

Afin que le pH de la formule contenant l'activateur de gaz présente une compatibilité optimale avec la peau, nous avons ajouté du citrate de sodium afin de créer un tampon acide citrique/citrate de sodium.

Une partie du tampon acide citrique/citrate de sodium peut avantageusement être substituée par du disodium pyrophosphate et vice versa, suivant les teneurs citées à titre d'exemple dans le tableau I ci-dessous :

Tableau III : Les valeurs sont exprimées en pourcentage poids/poids par rapport au poids de chacune des deux formules intermédiaires

|  | E 1 | E2 | E 3 | E 4 | E 5 | E 6 | E 7 |
|---|---|---|---|---|---|---|---|
| Bicarbonate de sodium | 5% | 5% | 5% | 5% | 3% | 3% | 3% |
| Acide citrique | 3.5% | 1.75% | 1.4% | 0 | 2.1% | 1.05% | 0 |
| Citrate de Na | 2.7% | 1.3% | 1% | 0 | 1.6% | 1.15% | 0 |
| Disodium pyrophosphate | 0 | 6% | 7.2% | 12% | 0 | 3.6% | 7.2% |

Une partie du tampon l'acide citrique/citrate de sodium peut avantageusement être substituée par du sodium dihydrogénophosphate mono hydrate et vice versa suivant les teneurs citées à titre d'exemple dans le tableau IV ci-dessous :

Tableau IV : Les valeurs sont exprimées en pourcentage poids/poids par rapport au poids de chacune des deux formules intermédiaires.

|  | E 1 | E8 | E9 |
|---|---|---|---|
| Bicarbonate de Sodium | 5% | 5% | 5% |
| Acide citrique | 3.5% | 1.5% | 0 |
| Citrate de Na | 2.7% | 0.5% | 0 |
| Sodium dihydrogénophosphate monohydrate | 0 | 6.2% | 10% |

Dans un mode particulier, il a été déterminé que lorsque la quantité d'acide citrique est supérieure ou également à 1,4, la quantité de mousse est optimale lorsque le disodium pyrophosphate est présent dans la composition selon l'équation suivante :

-

$$[C]=2.4[B]-2.4[A]/0.7$$

Lorsque :

[C] = teneur en poids en disodium pyrophosphate dans la composition intermédiaire A
[A] = teneur en poids en acide citrique monohydrate dans la composition intermédiaire A
[B] = teneur en poids en bicarbonate de sodium dans la composition intermédiaire B

L'équation ci-dessus permet ainsi de calculer les teneurs optimales entre le bicarbonate de sodium l'acide citrique et le pyrophosphate de sodium.

**Revendications**

1. Composition auto-moussante contenant du peroxyde de benzoyle destinée à une application topique rincée, comprenant:

   - au moins une composition intermédiaire B comprenant un agent générateur de gaz choisi parmi le bicarbonate de sodium, le bicarbonate de potassium, le carbonate de sodium, le carbonate de potassium et leurs mélanges,
   - au moins une composition intermédiaire A comprenant un agent activateur de l'agent générateur de gaz choisi parmi un acide, un sel de polyacide partiellement salifié, une solution tampon d'acide faible et de sa base conjuguée, et les mélanges de ces composés, et
   - du peroxyde de benzoyle contenu dans l'une au moins desdites compositions intermédiaires A et B.

2. Composition selon la revendication précédente, **caractérisée en ce que** le peroxyde de benzoyle est contenu dans la composition intermédiaire A.

3. Composition selon l'une quelconque des revendications précédentes, **caractérisée en ce qu'**elle ne comprend pas de tensioactifs moussants, ou qu'elle contient de tels tensioactifs en une teneur inférieure ou égale à 1% en poids, par rapport au poids de la composition totale.

4. Composition selon l'une quelconque des revendications précédentes, **caractérisée en ce que** le gaz généré à partir de l'agent générateur de gaz est du dioxyde de carbone ($CO_2$).

5. Composition selon l'une quelconque des revendications précédentes, **caractérisée en ce que** l'agent générateur de gaz est le bicarbonate de sodium.

6. Composition selon l'une quelconque des revendications précédentes, **caractérisée en ce que** l'agent générateur de gaz est présent dans la composition intermédiaire B en une quantité allant de 1% à 10%, préférentiellement de 2% à 8% en poids, par rapport au poids de la composition intermédiaire B.

7. Composition selon l'une quelconque des revendications précédentes, **caractérisée en ce que** l'agent activateur de l'agent générateur de gaz est choisi parmi l'acide citrique, l'acide tartrique, l'acide malique, l'acide lactique, l'acide phosphorique et l'acide pyrophosphorique, et les sels de ces acides.

8. Composition selon la revendication précédente, **caractérisée en ce que** ledit agent activateur est choisi parmi :

   - un tampon acide citrique/citrate de sodium seul ;
   - l'acide phosphorique, le phosphate de sodium, le disodium pyrophosphate seuls ou en mélange avec un tampon acide citrique/ citrate de sodium.

9. Composition selon la revendication précédente, **caractérisée en ce que** l'agent activateur de l'agent générateur de gaz est un tampon acide citrique/ citrate de sodium seul ou en mélange avec du phosphate sodique et/ou du disodium pyrophosphate.

10. Composition selon l'une quelconque des revendications précédentes, **caractérisée en ce que** la composition intermédiaire A se présente sous forme d'une solution, d'un gel, ou d'une émulsion, et de préférence la composition intermédiaire A se présente sous forme d'un gel.

11. Composition selon l'une quelconque des revendications 1 à 10, pour son utilisation comme médicament par application topique sur la peau.

12. Kit ou contenant unique à plusieurs compartiments comprenant de manière séparée au moins deux compositions intermédiaires:

   - une composition intermédiaire A comprenant au moins un agent activateur de l'agent générateur de gaz, telle que définie dans l'une quelconque des revendications 1 à 10; et
   - une composition intermédiaire B comprenant au moins un agent générateur de gaz telle que définie dans l'une quelconque des revendications 1 à 10 ;
   - du peroxyde de benzoyle étant contenu dans l'une au moins desdites compositions intermédiaires A et B.

**13.** Kit ou contenant unique à plusieurs compartiments selon la revendication 12, **caractérisé en ce qu'**il est conçu pour mélanger les compositions intermédiaires A et B en un rapport en poids A/B allant de 0,5 à 2, préférentiellement de 0,5 à 1,5, plus préférentiellement de 0,9 à 1,1, et encore plus préférentiellement de 1.

**Patentansprüche**

**1.** Selbstschäumende Zusammensetzung, die Benzoylperoxid enthält, zur topischen Anwendung mit Ausspülen, die Folgendes umfasst:

- mindestens eine Zwischenzusammensetzung B, die ein gaserzeugendes Mittel, ausgewählt aus Natriumbicarbonat, Kaliumbicarbonat, Natriumcarbonat, Kaliumcarbonat und deren Gemischen, umfasst,
- mindestens eine Zwischenzusammensetzung A, die ein Aktivierungsmittel für das gaserzeugende Mittel, ausgewählt aus einer Säure, einem partiell versalzten Polysäuresalz, einer Pufferlösung aus einer schwachen Säure und deren konjugierter Base und den Gemischen dieser Verbindungen, umfasst, und
- Benzoylperoxid, das in mindestens einer der Zwischenzusammensetzungen A und B enthalten ist.

**2.** Zusammensetzung nach dem vorhergehenden Anspruch, **dadurch gekennzeichnet, dass** das Benzoylperoxid in der Zwischenzusammensetzung A enthalten ist.

**3.** Zusammensetzung nach einem der vorhergehenden Ansprüche, **dadurch gekennzeichnet, dass** sie keine schäumenden Tenside umfasst oder dass sie solche Tenside in einem Gehalt von weniger als oder gleich 1 Gew.-%, bezogen auf das Gewicht der Gesamtzusammensetzung, umfasst.

**4.** Zusammensetzung nach einem der vorhergehenden Ansprüche, **dadurch gekennzeichnet, dass** das Gas, das aus dem gaserzeugenden Mittel erzeugt wird, Kohlendioxid ($CO_2$) ist.

**5.** Zusammensetzung nach einem der vorhergehenden Ansprüche, **dadurch gekennzeichnet, dass** das gaserzeugende Mittel Natriumbicarbonat ist.

**6.** Zusammensetzung nach einem der vorhergehenden Ansprüche, **dadurch gekennzeichnet, dass** das gaserzeugende Mittel in der Zwischenzusammensetzung B in einer Menge von 1 bis 10 Gew.-%, vorzugsweise von 2 bis 8 Gew.-%, bezogen auf das Gewicht der Zwischenzusammensetzung B, vorhanden ist.

**7.** Zusammensetzung nach einem der vorhergehenden Ansprüche, **dadurch gekennzeichnet, dass** das Aktivierungsmittel für das gaserzeugende Mittel aus Citronensäure, Weinsäure, Äpfelsäure, Milchsäure, Phosphorsäure und Pyrophosphorsäure und den Salzen dieser Säuren ausgewählt ist.

**8.** Zusammensetzung nach dem vorhergehenden Anspruch, **dadurch gekennzeichnet, dass** das Aktivierungsmittel aus den Folgenden ausgewählt ist:

- einem Citronensäure/Natriumcitrat-Puffer allein,
- Phosphorsäure, Natriumphosphat, Dinatriumpyrophosphat allein oder im Gemisch mit einem Citronensäure/Natriumcitrat-Puffer.

**9.** Zusammensetzung nach dem vorhergehenden Anspruch, **dadurch gekennzeichnet, dass** das Aktivierungsmittel für das gaserzeugende Mittel ein Citronensäure/Natriumcitrat-Puffer allein oder im Gemisch mit Natriumphosphat und/oder Dinatriumpyrophosphat ist.

**10.** Zusammensetzung nach einem der vorhergehenden Ansprüche, **dadurch gekennzeichnet, dass** die Zwischenzusammensetzung A in Form einer Lösung, eines Gels oder einer Emulsion vorliegt und vorzugsweise die Zwischenzusammensetzung A in Form eines Gels vorliegt.

**11.** Zusammensetzung nach einem der Ansprüche 1 bis 10 zur Verwendung als Medikament durch topische Anwendung auf die Haut.

**12.** Kit oder einzelner Behälter mit mehreren Fächern, das/der in getrennter Form mindestens zwei Zwischenzusammensetzungen umfasst:

- eine Zwischenzusammensetzung A, die mindestens ein Aktivierungsmittel für das gaserzeugende Mittel umfasst, nach einem der Ansprüche 1 bis 10 und
- eine Zwischenzusammensetzung B, die mindestens ein gaserzeugendes Mittel umfasst, nach einem der Ansprüche 1 bis 10,
- Benzoylperoxid, das in mindestens einer der Zwischenzusammensetzungen A und B enthalten ist.

13. Kit oder einzelner Behälter mit mehreren Fächern nach Anspruch 12, **dadurch gekennzeichnet, dass** es/er dafür ausgelegt ist, die Zwischenzusammensetzungen A und B in einem Gewichtsverhältnis A/B von 0,5 bis 2, vorzugsweise von 0,5 bis 1,5, stärker bevorzugt von 0,9 bis 1,1 und noch stärker bevorzugt von 1 zu mischen.

**Claims**

1. Self-foaming composition comprising benzoyl peroxide which is intended for a rinse-off topical application, comprising:

   - at least one intermediate composition B which comprises a gas-generating agent chosen from sodium bicarbonate, potassium bicarbonate, sodium carbonate, potassium carbonate and their mixtures,
   - at least one intermediate composition A which comprises an agent which activates the gas-generating agent chosen from an acid, a partially salified polyacid salt, a buffer solution of weak acid and of its conjugate base, and the mixtures of these compounds, and
   - benzoyl peroxide present in at least one of said intermediate compositions A and B.

2. Composition according to the preceding claim, **characterized in that** the benzoyl peroxide is present in the intermediate composition A.

3. Composition according to either one of the preceding claims, **characterized in that** it does not comprise foaming surfactants or that it comprises such surfactants in a content of less than or equal to 1% by weight, with respect to the weight of the total composition.

4. Composition according to any one of the preceding claims, **characterized in that** the gas generated from the gas-generating agent is carbon dioxide ($CO_2$).

5. Composition according to any one of the preceding claims, **characterized in that** the gas-generating agent is sodium bicarbonate.

6. Composition according to any one of the preceding Claims, **characterized in that** the gas-generating agent is present in the intermediate composition B in an amount ranging from 1% to 10% by weight, preferably from 2% to 8% by weight, with respect to the weight of the intermediate composition B.

7. Composition according to any one of the preceding claims, **characterized in that** the agent which activates the gas-generating agent is chosen from citric acid, tartaric acid, malic acid, lactic acid, phosphoric acid and pyrophosphoric acid, and the salts of these acids.

8. Composition according to the preceding claim, **characterized in that** said activating agent is chosen from:

   - a citric acid/sodium citrate buffer alone;
   - phosphoric acid, sodium phosphate, disodium pyrophosphate, which are used alone or as a mixture with a citric acid/sodium citrate buffer.

9. Composition according to the preceding claim, **characterized in that** the agent which activates the gas-generating agent is a citric acid/sodium citrate buffer, alone or as a mixture with sodium phosphate and/or disodium pyrophosphate.

10. Composition according to any one of the preceding claims, **characterized in that** the intermediate composition A is provided in the form of a solution, a gel or an emulsion, and preferably the intermediate composition A is provided in the form of a gel.

11. Composition according to any one of Claims 1 to 10, for use as medicament by topical application to the skin.

12. Kit or single multicompartment container separately comprising at least two intermediate compositions:

   - an intermediate composition A comprising at least one agent which activates the gas-generating agent, as defined in any one of Claims 1 to 10, and
   - an intermediate composition B comprising at least one gas-generating agent, as defined in any one of Claims 1 to 10;
   - benzoyl peroxide being present in at least one of said intermediate compositions A and B.

13. Kit or single multicompartment container according to Claim 12, **characterized in that** it is designed for mixing the intermediate compositions A and B in an A/B ratio by weight ranging from 0.5 to 2, preferably from 0.5 to 1.5, more preferably from 0.9 to 1.1 and more preferably still of 1.

# Fig. 1

Fig. 2